(19) Europäisches Patentamt
European Patent Office
Office européen des brevets

(11) EP 0 720 847 B1

(12) **FASCICULE DE BREVET EUROPEEN**

(45) Date de publication et mention de la délivrance du brevet:
**10.09.1997 Bulletin 1997/37**

(51) Int. Cl.$^6$: **A61K 7/48**, A61K 7/06

(21) Numéro de dépôt: **95402771.0**

(22) Date de dépôt: **11.12.1995**

# (54) Composition cosmétique ou dermatologique comprenant au moins un céramide 6

Eine mindestens ein Ceramid 6 enthaltende kosmetische oder dermatologische Zubereitung

Cosmetic or dermatological composition containing at least one ceramide 6

(84) Etats contractants désignés:
**AT BE CH DE DK ES FR GB GR IE IT LI LU MC NL PT SE**

(30) Priorité: **04.01.1995 FR 9500045**

(43) Date de publication de la demande:
**10.07.1996 Bulletin 1996/28**

(73) Titulaire: **L'OREAL**
**75008 Paris (FR)**

(72) Inventeurs:
- **Leveque, Jean-Luc**
**F-93340 Le Raincy (FR)**
- **Saint-Leger, Didier**
**F-92400 Courbevoie (FR)**

(74) Mandataire: **Tezier Herman, Béatrice**
**L'OREAL,**
**Département Propriété Industrielle,**
**90, rue du Gal Roguet**
**92583 Clichy Cédex (FR)**

(56) Documents cités:
**FR-A- 2 679 770** **FR-A- 2 700 953**

- **PATENT ABSTRACTS OF JAPAN vol. 11 no. 115 (C-415) [2562] ,10 Avril 1987 & JP-A-61 260008 (SUNSTAR INC.) 18 Novembre 1986,**
- **ARCHIVES OF DERMATOLOGY, vol. 123, no. 10, Octobre 1987 pages 1361-1363, PHILIP W. WERTZ ET AL**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 720 847 B1

Printed by Rank Xerox (UK) Business Services
2.14.14/3.4

## Description

L'invention concerne une composition cosmétique ou dermatologique qui comprend, à titre d'agent principal de réduction de la perte en eau de la peau et/ou des fibres kératiniques, au moins un céramide 6.

L'exposition de la peau et des cheveux au froid, au soleil, aux atmosphères de faible humidité relative, les traitements répétées avec des compositions de lavage ou encore le contact avec des solvants organiques sont des facteurs qui entraînent, à des degrés divers, un déssèchement apparent. La peau apparaît plus sèche, moins souple et le relief cutané plus prononcé. Par ailleurs, les cheveux, qui sont soumis fréquemment à certains traitements capillaires, perdent leur aspect brillant et peuvent devenir rèches et cassants.

La Demanderesse a donc recherché à formuler des compositions qui permettent de prévenir ou de corriger ces phénomènes se traduisant par un déssèchement apparent et qui donnent ou redonnent à la peau sa souplesse et aux cheveux leur brillance et leur douceur.

Pour résoudre ce problème, on a déjà proposé d'utiliser des céramides (voir FR-A-2 679 770). On sait, en effet, On sait, en effet, que ces composés sont les éléments constitutifs prépondérants des lipides intercornéocytaires du stratum corneum et participent au maintien de l'intégrité de la barrière cutanée.

Lorsqu'ils proviennent d'extraits naturels, les céramides utilisés en cosmétique sont toujours des mélanges de différents types de céramides, leur teneurs et leurs natures dépendant notamment de leur provenance et de leur procédé d'extraction.

Le présente invention repose sur la découverte que ce sont, parmi les céramides, les céramides 6 qui permettent le plus d'influencer le phénomène de réduction de la perte en eau et qui permettent donc de maintenir ou de reproduire la fonction barrière de la peau de manière significative.

Ainsi, la présente invention a pour objet une composition cosmétique ou dermatologique, caractérisée en ce qu'elle comprend au moins un céramide 6 à titre d'agent principal de réduction de la perte en eau de la peau et/ou des fibres kératiniques.

Elle a également pour objet l'utilisation de céramides 6 dans une composition cosmétique ou dermatologique en tant qu'agent principal de réduction de la perte en eau de la peau et/ou des fibres kératiniques.

Elle a enfin pour objet un procédé de traitement cosmétique de la peau ou des fibres kératiniques (cheveux ou poils) consistant à appliquer sur ces derniers une composition selon l'invention.

De préférence, la présente invention s'applique à la peau.

Les céramides de types 1 à 6 sont décrits par DOWNING dans Arch. Dermatol, Vol. 123, 1381-1384, 1987. Les céramides de types 1 à 6 ont la structure suivante :

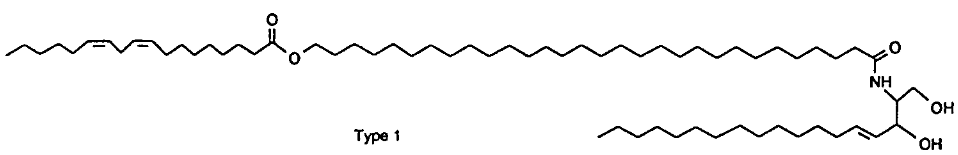

Type 1

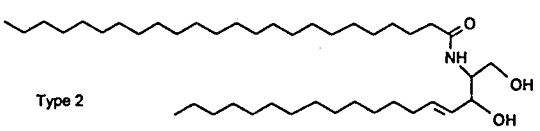

Type 2

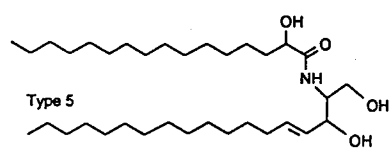

Type 5

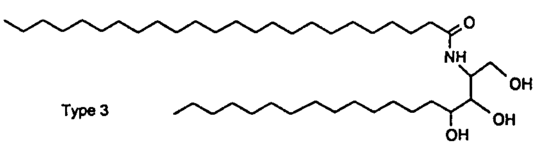

Type 3

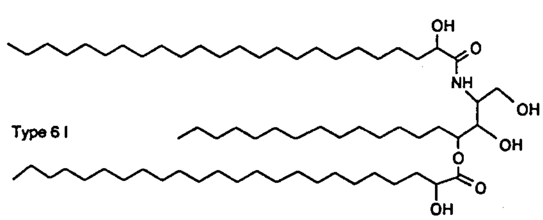

Type 6 I

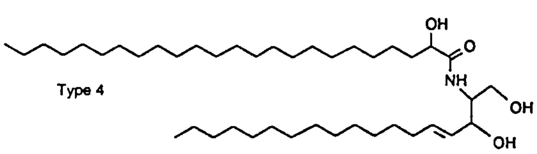

Type 4

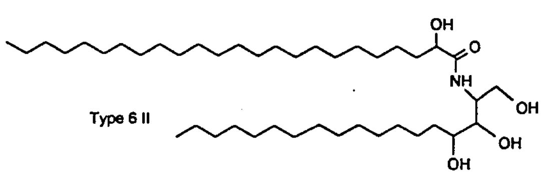

Type 6 II

Ainsi, les céramides 6 sont des phytosphingosines de deux types : 6I ou 6II.

Les céramides 6 utilisés dans la présente invention peuvent être des céramides d'origine naturelle.

Dans la présente invention, on utilise le céramide 6I ou 6II seuls ou en mélange, le mélange de céramides 6 provenant généralement de l'extraction de ceux-ci à partir du mélange de céramides présent dans le stratum corneum.

Les céramides 6, utilisés dans la présente invention, peuvent être obtenus par extraction du stratum corneum de la peau de mammifères, par toute méthode classique connue en soi, suivie d'une délipidation progressive et sélective, notamment par des solvants appropriés.

La peau de mammifères peut être de la peau d'homme, de porc, de bovins, de cheval, de mouton, de chèvre, de souris, de rat, de lapin, de chien, de cobaye, de chat, de singe et similaires. Le stratum corneum est isolé de la peau par des méthodes classiques physicochimiques. Ainsi, la peau prélevée peut être chauffée à des températures voisines de 60°C, puis traitée à la trypsine.

La délipidation progressive et sélective permettant d'isoler les céramides 6 est généralement réalisée en immergeant le stratum corneum dans un ou plusieurs solvants appropriés, tels que notamment l'hexane, l'acétone, le méthanol, l'éthanol, le chloroforme et l'éther éthylique. Ainsi, on préfère immerger le stratum corneum pendant environ 30 minutes dans de l'hexane, puis pendant environ 60 minutes dans de l'acétone et enfin dans un mélange chloroforme/méthanol pendant environ 120 minutes.

Par agent principal de réduction de la perte en eau, on entend selon l'invention que le pourcentage minimal en poids de céramide 6 présent dans la composition par rapport aux céramides totaux présents en tant qu'agents de réduction de la perte en eau dans la composition est plus élevé que son pourcentage en poids par rapport au mélange naturel de céramides totaux dont il peut provenir.

Selon l'analyse quantitative par chromatographie sur couche mince faite par Wertz, Miethke, Long et coll., la répartition en pourcentage en poids des céramides présents dans l'épiderme est la suivante (J. Invest. Dermatol., 1985, 84, 410-412) :

| Céramides de types | Pourcentages |
|---|---|
| 1 | 7,0 +/- 3,2 |
| 2 | 21,0 +/- 4,9 |
| 3 | 13,4 +/- 4,3 |
| 4/5 | 22,2 +/- 4,5 |
| 6I | 9,8 +/- 1,1 |
| 6II | 13,6 +/- 4,5 |

Ainsi, selon cette analyse et pour donner un ordre de grandeur, le pourcentage en poids des céramides 6I dans la composition est supérieur à 9,8 % (par rapport au poids total des céramides présents en tant qu'agent de réduction de la perte en eau dans la composition) et/ou le pourcentage en poids des céramides 611 dans la composition est supérieur à 13,6 % (par rapport au poids total des céramides présents en tant qu'agent de réduction de la perte en eau dans la composition). De préférence, le pourcentage des céramides 6 présents dans la composition selon l'invention est supérieur à 23,4 % (par rapport au poids total des céramides présents en tant qu'agent de réduction de la perte en eau dans la composition). Avantageusement, le pourcentage minimal en poids des céramides 6 présents dans la composition selon l'invention est supérieur à 35%. Bien entendu, ce pourcentage peut être plus élevé et peut atteindre 100%, le seul agent de réduction de la perte en eau présent dans la compostion étant alors les céramides 6.

Les compositions selon l'invention peuvent se présenter sous forme d'émulsions (lait ou crème), de lotions hydroalcooliques, huileuses ou oléoalcooliques, de gels, de dispersions ou de bâtonnets solides, de sprays ou de mousses aérosols.

Selon l'invention, les céramides 6 représentent généralement de 0,01% à 20%, et de préférence 0,05 à 10% du poids total de la composition.

Les compositions sont par exemple des lotions, des laits ou crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings ou des mascaras.

Ces compositions peuvent également se présenter sous la forme de bâtons pour les lèvres destinés soit à les colorer, soit à éviter les gerçures, ou des produits de maquillage pour les yeux ou de fards et fonds de teint pour le visage.

Lorsque les compositions selon l'invention se présentent sous forme d'émulsions du type eau dans huile ou huile dans eau, la phase grasse est essentiellement constituée d'un mélange de céramides 6 et d'au moins une huile ou un corps gras.

La phase grasse des émulsions peut constituer 5 à 60% du poids total de l'émulsion.

La phase aqueuse desdites émulsions constitue de préférence 30 à 85% du poids total de l'émulsion.

La proportion de l'agent émulsionnant peut être comprise entre 0,1 et 20 %, et de préférence entre 1 et 12% du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau; les lotions huileuses peuvent en outre constituer des huiles moussantes contenant une agent tensio-actif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acides gras tels que les triglycérides d'acides gras ayant de 6 à 18 atomes de carbone, les alcools gras; les émulsionnants comme les alcools gras oxyéthylénés ou les alcyléthers de polyglycérol; les solvants tels que les monoalcools ou polyalcools inférieurs contenant de 1 à 6 atomes de carbone ou encore l'eau.

Les mono- ou polyalcools plus particulièrement préférés sont choisis parmi l'éthanol, l'isopropanol, le propylèneglycol, le glycérol ou le sorbitol.

A titre de corps gras, parmi les huiles minérales, on peut citer l'huile de vaseline; parmi les huiles animales, les huiles de baleine, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germe de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en C12 à C22 saturés ou insaturés et d'alcools inférieurs comme l'isopropanol ou le glycérol ou d'alcools gras en C8 à C22, linéaires ou ramifiés, saturés ou insaturés ou encore d'alcanediols-1,2 en C10-C22.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C12-C18 comportant de 2 à 10 moles de glycérol.

Il peut être aussi utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermopharmacie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de céramides 6 dans l'eau en présence de tensio-actif ou encore de dispersions aqueuses de sphérules lipidiques, constituées de couches moléculaires organisées enfermant une phase aqueuse encapsulée, ces couches étant constituées d'au moins un céramide 6 associé à au moins un autre composé lipidique.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991 , 1 477 048 et 2 091 516 ou dans la demande de brevet international WO 83/01 571.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le

trihexosylcéramide, ainsi que les phospholipides tels que le phosphatidylglycérol et le phosphatidylinositol.

La phase continue de la dispersion qui entoure les sphérules est une phase aqueuse.

Les sphérules en dispersion ont généralement un diamètre compris entre 0,05 μm et 5 μm.

La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet français 2 315 991 de la Demanderesse, selon lequel on prépare une dispersion de sphérules constituées de couches moléculaires organisées renfermant une phase aqueuse à encapsuler, en mettant en contact d'une part des céramides 6 associées à un ou plusieurs lipide(s) défini(s) ci-dessus et d'autre part la phase aqueuse à encapsuler dans les sphérules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.

Le rapport pondéral entre la phase aqueuse à encapsuler et les céramides 6 associés aux lipides formant la phase lamellaire est de préférence compris entre 0,1 et 20.

Le rapport pondéral de la phase aqueuse de dispersion que l'on ajoute à la phase lamellaire que l'on disperse est de préférence compris entre 2 et 100, la phase de dispersion et la phase aqueuse à encapsuler étant de préférence isoosmotiques.

L'agitation est réalisée au moyen d'un agitateur à secousses. Le procédé est de préférence mis en oeuvre à une température comprise entre 30° et 120 ° C.

Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n° 9, pages 4194-4198 (1978), par SZOKA et PAPAHADJOPOULOS.

On peut également mettre en oeuvre le procédé qui comprend la succession d'étapes consistant à dissoudre au moins un lipide dans au moins un solvant organique non miscible à l'eau ; ajouter la phase organique ainsi obtenue à une phase aqueuse ; former une dispersion des deux phases sous forte agitation, la taille des vésicules pouvant être réglée en faisant varier la vitesse d'agitation au cours de ce mélange de phase ; conduire l'évaporation du (ou des) solvant(s) sous forte agitation ; et, le cas échéant, concentrer la dispersion.

Les substances actives peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules.

Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les y-dialdéhydes tels que l'aldéhyde tartrique, ces composés étant éventuellement associés à des colorants ; des filtres solaires hydrosolubles; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou antitumoraux.

Lorsque les substances actives sont liposolubles, elles se trouvent incorporées dans les feuillets des vésicules. Elle peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

On peut également ajouter à la phase aqueuse des dispersions de sphérules une phase liquide L non miscible à l'eau. En particulier, la composition selon l'invention peut contenir de 2 à 70 % en poids de phase liquide L, non miscible à l'eau, par rapport au poids total de la composition, la proportion pondérale relative de(s) lipide(s) constitutif(s) de vésicules par rapport à la phase liquide dispersée, L étant comprise entre 0,02/1 et 10/1.

Le(s) constituants de la phase liquide L dispersée dans la phase aqueuse D, peut (peuvent) être choisi(s) dans le groupe formé par les huiles, telles que les esters d'acides gras et de polyols et les esters d'acide gras et d'alcools ramifiés de formule $R^7$-COOR$^8$, formule dans laquelle $R^7$ représente le reste d'un acide gras supérieur comportant de 7 à 19 atomes de carbone et $R^8$ représente une chaîne hydrocarbonée ramifiée contenant de 3 à 20 atomes de carbone ; les hydrocarbures, tels que l'hexadécane, l'huile de paraffine, le perhydrosqualène ; les hydrocarbures halogénés, tels que le perfluorodécahydronaphtalène ; la perfluorotributylamine ; les polysiloxanes ; les esters d'acides organiques, les éthers et polyéthers. La phase liquide L peut renfermer au moins un parfum et/ou au moins une substance active liposoluble. De telles substances liposolubles peuvent être constituées par les filtres solaires liposolubles, les substances destinées à améliorer l'état des peaux sèches ou séniles, les tocophérols, les vitamines E ou F, la vitamine A et ses

esters, l'acide rétinoïque, les antioxydants, les acides gras essentiels, l'acide glycyrrhétinique, les agents kératolytiques et les caroténoïdes.

On peut également ajouter aux dispersions de sphérules divers adjuvants tels que des opacifiants, des gélifiants, des arômes, des parfums ou des colorants.

Les dispersions de sphérules lipidiques présentent l'intérêt de véhiculer des substances actives qui se trouvent ainsi masquées et protégées vis-à-vis des différents agents d'altération : oxydants et plus généralement composés réactifs vis-à-vis des substances actives encapsulées. La pénétration et la fixation des substances actives peuvent être modulées par la variation de la taille des sphérules et de leur charge électrique. L'action de ses substances active peut également être ainsi différée (effet retard). Enfin, il est possible d'obtenir grâce à l'utilisation des céramides 6 et de subtances actives combinées une action bénéfique spécifique de la substance active utilisée et en même temps assouplissante, particulièrement intéressante dans le cas du traitement de la peau.

L'invention a enfin pour objet un procédé de traitement cosmétique de la peau ou des fibres kératiniques, caractérisé en ce qu'il consiste à appliquer sur la peau ou sur les fibres kératiniques une composition telle que définie ci-dessus. La composition selon l'invention étant bien adaptée à l'hydratation de la peau, le procédé de traitement cosmétique de la peau est plus particulièrement destiné à hydrater la peau.

Toutefois, la composition de l'invention peut aussi être utilisée dans le traitement des xéroses et dans tout traitement de la peau où il est nécessaire de protéger la peau.

L'invention a aussi pour objet l'utilisation de la composition définie précédemment pour préparer une pommade ou un onguent destiné au traitement thérapeutique des peaux sèches.

Les exemples qui suivent sont donnés à titre illustratif et non limitatif. Les pourcentages des exemples sont donnés en poids.

Exemple 1 :

Le stratum cornéum est isolé de la peau par la méthode suivante : chaleur à 58°C, puis trypsine. Les échantillons sont découpées à l'emporte-pièce (disques de 10mm), identifiés et pesés avant d'être montés dans les cuves de perte en eau.

La mesure de la perte insensible en eau (PIE) se fait à l'aide d'un évaporimètre qui est un appareil qui détermine quantitativement une évaporation d'eau, c'est à dire un transport d'eau par diffusion, à partir ou vers des surfaces en contact avec l'atmosphère.

Le transport de l'eau par diffusion proche d'une surface (distance inférieure à 1cm)) est déterminée par la loi de Fick :

$$1/A \ . \ dm/dt = - D \ dp/dx$$

où

A = aire de la surface ($m^2$)
m = poids de l'eau transportée (g)
t = temps
D =

$$0{,}0877 \ gm^{-1}h^{-1} \left(\frac{10^5}{760} Pa\right)^{-1} \left(gm^{-1}h^{-1}(mmHg)^{-1}\right)$$

constante relative au coefficient de diffusion

p = pression partielle de la vapeur d'eau dans l'air

$$\left(\frac{10^5}{760} Pa \ (mmHg)\right)$$

X = distance à partir de la surface (m)

Cette formule indique que le taux d'évaporation dm/dt est proportionnel au gradient de pression partielle dp/dx et

qu'il peut donc être déterminé par la mesure de ce gradient.

L'évaporimètre emploie une association de deux capteurs. Une petite surface de la peau est délimitée par une capsule cylindrique en téflon. Le but de cette capsule est de protéger la surface de mesure des courants d'air. A deux points A et B, disposés à des distances différentes, est placé une paire de transducteurs, l'un étant un condensateur pour la mesure de l'humidité relative, l'autre une thermistance pour la mesure de la température.

A partir des signaux recueillis par ce transducteur, l'instrument calcule d'abord la pression partielle de la vapeur d'eau aux deux points A et B puis le gradient de pression partielle et finalement le taux d'évaporation (PIE).

Selon cette méthode de mesure (mesure effectuée avec un appareil Servomed®), on a déterminé le PIE du stratum cornéum prélevé à différents stades de délipidation.

La délipidation progressive et sélective permettant d'isoler les céramides 6 est réalisée en immergeant le stratum corneum pendant environ 30 minutes dans de l'hexane, puis pendant environ 60 minutes dans de l'acétone et enfin dans un mélange chloroforme/méthanol pendant environ 120 minutes.

L'analyse des lipides extraits est réalisée par chromatographie sur couche mince.

Les résultats obtenus sont rassemblés dans le tableau (1).

Tableau (1)

| | 30 minutes hexane | 30 minutes hexane | 60 minutes acétone | 120 minutes chloroforme/méthanol (2/1) |
|---|---|---|---|---|
| esters de stérol | 9,8 ± 1,6 | 13,3 ± 2,8 | 9,5 ± 3,5 | 4,4 ± 1,6 |
| triglycérides | 51 ± 7 | 43,4 ± 5,5 | 32 ± 6 | 12,3 ± 1,8 |
| acides gras libres | 9,8 ± 1,7 | 15,3 ± 2,3 | 14 ± 2 | 14,3 ± 1,6 |
| cholestérol | 16,7 ± 2,4 | 17 ± 3 | 16,8 ± 1,4 | 15,5 ± 1,3 |
| céramide 1 | 0,31 ± 0,4 | 0,51 ± 0,6 | 0,16 ± 0,15 | 1,58 ± 0,09 |
| céramide 2 | 4,45 ± 2,4 | 6,4 ± 2,5 | 5,2 ± 4 | 7,5 ± 3 |
| céramide 3 | 5,1 ± 6 | 2,9 ± 3 | 2,8 ± 1,5 | 5,3 ± 3 |
| céramide 4 | 1,17 ± 1,4 | 0,11 ± 0,02 | 4,5 ± 2 | 10,4 ± 1 |
| céramide 5 | 0,32 ± 0,3 | 0,11 ± 0,02 | 5,6 ± 2,5 | 8,2 ± 0,6 |
| céramide 6 | 0,22 ± 0,4 | 0,11 ± 0,02 | 1,5 ± 2 | 12,4 ± 1,3 |
| sulfate de cholestérol | 0,96 ± 1 | 0,64 ± 0,8 | 7,75 ± 5 | 7,7 ± 1,3 |
| masse totale (µg) | 125 ± 13 | 30,3 ± 8 | 33 ± 9 | 75 ± 11 |
| PIE (g/m$^2$.H) | 1,34 ± 0,3 | 1,34 ± 0,4 | 1,40 ± 0,5 | 4,81 ± 0,9 |

Les résultats sont exprimés en pourcentage en poids sauf menton contraire.

La PIE initiale, c'est à dire avant toute étape de délipidation, est de 1,14 ± 0,3 g/m$^2$.H.

Ces résultats montrent que la plus grande des variations en PIE a lieu lorsque est extraite la plus grande proportion de céramides 6, c'est à dire après l'extraction pendant 120 minutes avec un mélange chloroforme et méthanol. Avant ces 120 minutes et après le traitement à l'acétone, c'est le céramide 6 qui reste en plus grande quantité : 92%.

Les céramides 6 ainsi extraits peuvent être formulés tels que décrits dans les exemples suivants.

**Exemple 2 : Emulsion huile-dans-eau hydratante**

| | |
|---|---|
| - Silicone volatile 7158 de chez Union Carbide | 10,0 |
| - Perhydrosqualène | 18,0 |
| - Huile de vaseline | 5,0 |
| - Lanoline liquide | 4,0 |
| - Arlacel 165 de chez Atlas | 6,0 |
| - Tween 60 de chez Atlas | 2,0 |
| - Céramides 6 | 0,88 |
| - Alcool cétylique | 1,2 |
| - Alcool stéarique | 2,5 |
| - Hydroxyde de sodium | 0,008 |
| - Propylène glycol | 5,0 |
| - Triéthanolamine | 0,1 |
| - Conservateur | 0,3 |
| - Anti-oxydant | 0,3 |
| - Eau déminéralisée | qsp 100 |

L'émulsion se présente sous la forme d'une crème blanche à appliquer le soir pour réparer et hydrater la peau. Elle est destinée à toutes les peaux.

**Exemple 3: Emulsion huile-dans-eau hydratante**

| | |
|---|---|
| - Huile de germe de maïs | 2,0 |
| - Monostéarate de glycérol | 3,0 |
| - PEG 400 | 3,0 |
| - Carbopol 941 | 0,2 |
| - Myristate d'isopropyle | 1,0 |
| - Céramides 6 | 0,3 |
| - Alcool cétylique | 3,0 |
| - Alcool stéarique | 3,0 |
| - Hydroxyde de sodium | 0,008 |
| - Propylène glycol | 5,0 |
| - Conservateur | 0,3 |
| - Parfum | 0,5 |
| - Eau déminéralisée | qsp 100 |

Cette émulsion est une crème blanche de jour, hydratante, utilisable pour tous types peaux.

**Exemple 4 : Emulsion eau-dans-huile hydratante**

| | |
|---|---|
| - Huile de vaseline | 10,0 |
| - Protegin X de chez Goldschmidt | 20,0 |
| - Huile de tournesol | 15,0 |
| - Composition aromatique | 1,0 |
| - Céramides 6 | 0,1 |
| - Sulfate de magnésium | 0,5 |
| - Glycérol | 5,0 |
| - Cétrol HE de chez Henkel | 4,0 |
| - Conservateur | 0,3 |
| - Eau déminéralisée | qsp 100 |

Cette crème est plus spécialement destinée au traitement de nuit des peaux sèches sensibles.

**Exemple 5 : Emulsion eau-dans-huile hydratante**

| | |
|---|---|
| - Abil We 09 de chez Goldschmidt | 5,0 |
| - Myristate d'isopropyle | 5,0 |
| - Silicone volatile 7158 d'Union Carbide | 8,0 |
| - Aérosil R 812 de chez Dégussa | 0,4 |
| - Huile de Purcellin de chez Dragocco | 14,0 |
| - Chlorure de sodium | 0,5 |
| - Transcutol de chez Gattefosse | 3,0 |
| - Céramides | 2,9 |
| - Hydroxyde de sodium | 0,008 |
| - Huile de vaseline | 5,0 |
| - Conservateur | 0,3 |
| - Eau déminéralisée | qsp 100 |

Cette crème est plus spécialement destinée au traitement de nuit des peaux sèches.

**Exemple 6: Gel hydroalcoolique**

| | |
|---|---|
| - Carbopol 940 | 0,9 |
| - Céramides 6 | 0,3 |
| - Alcool éthylique | 20,0 |
| - Triéthanolamine | 0,3 |
| - Propylène glycol | 5,0 |
| - Transcutol | 5,0 |
| - Conservateur | 0,3 |
| - Parfum | 0,3 |
| - Eau déminéralisée | qsp 100 |

Ce gel est destiné à l'hydratation et restructuration des peaux sèches.

**Exemple 7 : Gel émulsionné huile-dans-eau**

| | |
|---|---|
| - Carbopol 940 | 0, 6 |
| - Silicone volatile 7158 d'Union Carbide | 3,0 |
| - Huile de Purcellin de chez Dragocco | 7,0 |
| - Céramides 6 | 0,1 |
| - Alcool éthylique | 10,0 |
| - Triéthanolamine | 0,2 |
| - Tefosse 63 de chez Gattefosse | 3,0 |
| - Cétiol HE | 2,0 |
| - Caféine | 1,0 |
| - Conservateur | 0,3 |
| - Parfum | 0,4 |
| - Eau déminéralisée | qsp 100 |

Ce gel est destiné à l'hydratation du corps de tout type de peau.

**Exemple 8 : Gel aqueux**

| | |
|---|---|
| - Carbopol 940 | 0,6 |
| - Transcutol | 5,0 |
| - Triéthanolamine | 0,3 |
| - Conservateur | 0,3 |
| - Propylène glycol | 3,0 |
| - Hydroxyde de sodium | 0,007 |
| - Céramides 6 | 0,1 |

Ce gel est plutôt destiné àl'hydratation du corps des peaux sensibles.

**Exemple 9 : Crème aux liposomes non ioniques**

| | |
|---|---|
| - Carbopol 940 | 0,2 |
| - Transcutol | 3,0 |
| - Triéthanolamine | 0,2 |
| - Conservateur | 0,3 |
| - Polyglycéryl-3-cétyl éther | 3,8 |
| - B-sitostérol | 3,8 |
| - Dicétyl-phosphate | 0,4 |
| - Hydroxyde de sodium | 0,007 |
| - Céramides 6 | 0,55 |
| - Huile de tounesol | 35,0 |
| - Parfum | 0,6 |
| - Eau déminéralisée | qsp 100 |

Ce gel est plutôt destiné à l'hydratation et la restructuration des peaux sèches, notamment pendant la nuit.

**Revendications**

**1.** Composition cosmétique ou dermatologique, caractérisée en ce qu'elle comprend au moins un céramide 6 à titre d'agent principal de réduction de la perte en eau de la peau et/ou des fibres kératiniques.

**2.** Composition selon la revendication 1, caractérisée en ce que le céramide 6 utilisé est d'origine naturelle.

**3.** Composition selon l'une des revendications 1 ou 2, caractérisée en ce que le pourcentage en poids des céramides 61 présents est supérieur à 9,8 % par rapport aux céramides présents en tant qu'agents de réduction de la perte en eau dans la composition.

**4.** Composition selon l'une quelconque des revendications précédentes, caractérisée en ce que le pourcentage en poids en céramides 6ll est supérieur à 13,6 % par rapport aux céramides présents en tant qu'agents de réduction de la perte en eau dans la composition.

**5.** Composition selon l'une quelconque des revendications précédentes, caractérisée en ce que le pourcentage en poids en céramides 6 est supérieur à 23,4 % par rapport aux céramides présents en tant qu'agents de réduction de la perte en eau dans la composition.

**6.** Composition selon la revendication précédente, caractérisée en ce que le pourcentage en poids en céramides 6 est supérieur à 35 % par rapport aux céramides présents en tant qu'agents de réduction de la perte en eau dans la composition.

**7.** Composition selon l'une quelconque des revendications précédentes, caractérisée en ce qu'elle se présente sous forme d'émulsions, de lotions hydroalcooliques, huileuses ou oléoalcooliques, de gels, de dispersions ou de bâtonnets solides, de sprays ou de mousses aérosols.

**8.** Composition selon l'une quelconque des revendications précédentes, caractérisée en ce que les céramides 6 représentent de 0,01% à 20%, et de préférence 0,05 à 10% du poids total de la composition.

**9.** Procédé de traitement cosmétique de la peau ou des fibres kératiniques, caractérisé en ce qu'il consiste à appliquer sur la peau ou sur les fibres kératiniques une composition selon l'une quelconque des revendications précédentes.

**10.** Utilisation de la composition selon l'une quelconque des revendications 1 à 8 pour préparer une pommade ou un

onguent destiné au traitement thérapeutique des peaux sèches.

11. Utilisation de céramides 6 dans une composition cosmétique ou dermatocosmétique en tant qu'agent principal de réduction de la perte en eau de la peau et/ou des fibres kératiniques.

**Claims**

1. Cosmetic or dermatological composition, characterized in that it comprises at least one ceramide 6 as main agent for reducing the loss of water from the skin and/or from keratin fibres.

2. Composition according to Claim 1, characterized in that the ceramide 6 used is of natural origin.

3. Composition according to either of Claims 1 or 2, characterized in that the percentage by weight of ceramides 61 present is greater than 9.8% relative to the ceramides present as agents for reducing the water loss in the composition.

4. Composition according to any one of the preceding claims, characterized in that the percentage by weight of ceramides 611 is greater than 13.6% relative to the ceramides present as agents for reducing the water loss in the composition.

5. Composition according to any one of the preceding claims, characterized in that the percentage by weight of ceramides 6 is greater than 23.4% relative to the ceramides present as agents for reducing the water loss in the composition.

6. Composition according to the preceding claim, characterized in that the percentage by weight of ceramides 6 is greater than 35% relative to the ceramides present as agents for reducing the water loss in the composition.

7. Composition according to any one of the preceding claims, characterized in that it is in the form of an emulsion, an aqueous-alcoholic, oily or oleo-alcoholic lotion, a gel, a dispersion or a solid stick, a spray or an aerosol foam.

8. Composition according to any one of the preceding claims, characterized in that the ceramides 6 represent from 0.01% to 20%, and preferably 0.05 to 10%, of the total weight of the composition.

9. Process for the cosmetic treatment of the skin or of keratin fibres, characterized in that it consists in applying a composition according to any one of the preceding claims to the skin or keratin fibres.

10. Use of the composition according to any one of Claims 1 to 8, for preparing a salve or an ointment intended for the therapeutic treatment of dry skin.

11. Use of ceramides 6 in a cosmetic or dermatocosmetic composition as main agent for reducing the loss of water from the skin and/or from keratin fibres.

**Patentansprüche**

1. Kosmetische oder dermatologische Zusammensetzung, dadurch gekennzeichnet, daß sie mindestens ein Ceramid 6 als Hauptwirkstoff zur Verringerung des Wasserverlustes der Haut und/oder der Keratinfasern enthält.

2. Zusammensetzung nach Anspruch 1, dadurch gekennzeichnet, daß das verwendete Ceramid 6 natürlichen Ursprungs ist.

3. Zusammensetzung nach einem der Ansprüche 1 oder 2, dadurch gekennzeichnet, daß der gewichtsprozentuale Anteil der Ceramide 6I mehr als 9,8%, bezogen auf die in der Zusammensetzung als Mittel zur Verringerung des Wasserverlustes vorhandenen Ceramide, beträgt.

4. Zusammensetzung nach einem der vorstehenden Ansprüche, dadurch gekennzeichnet, daß der gewichtsprozentuale Anteil der Ceramide 6II mehr als 13,6%, bezogen auf die in der Zusammensetzung als Mittel zur Verringerung des Wasserverlustes vorhandenen Ceramide, beträgt.

5. Zusammensetzung nach einem der vorstehenden Ansprüche, dadurch gekennzeichnet, daß der gewichtsprozentuale Anteil der Ceramide 6 mehr als 23,4 Gew.-%, bezogen auf die in der Zusammensetzung als Mittel zur Verringerung des Wasserverlustes vorhandenen Ceramide, beträgt.

6. Zusammensetzung nach dem vorstehenden Anspruch, dadurch gekennzeichnet, daß der gewichtsprozentuale Anteil der Ceramide 6 mehr als 35 Gew.-%, bezogen auf die in der Zusammensetzung als Mittel zur Verringerung des Wasserverlustes vorhandenen Ceramide, beträgt.

7. Zusammensetzung nach einem der vorstehenden Ansprüche, dadurch gekennzeichnet, daß sie in der Form von Emulsionen, von hydroalkoholischen, öligen oder oleoalkoholischen Lotionen, von Gelen, von Dispersionen, von festen Stäbchen, von Sprühmitteln oder von Aerosolschäumen vorliegt.

8. Zusammensetzung nach einem der vorstehenden Ansprüche, dadurch gekennzeichnet, daß die Ceramide 6 insgesamt 0,01 bis 20 Gew.-% und vorzugsweise 0,05 bis 10 Gew.-% der Zusammensetzung ausmachen.

9. Verfahren zur kosmetischen Behandlung von Haut oder Keratinfasern, dadurch gekennzeichnet, daß es darin besteht, daß man auf die Haut oder die Keratinfasern eine Zusammensetzung nach einem der vorstehenden Ansprüche aufbringt.

10. Verwendung der Zusammensetzung nach einem der Ansprüche 1 bis 8 zur Herstellung einer Pomade oder einer Salbe zur therapeutischen Behandlung von trockener Haut.

11. Verwendung von Ceramiden 6 in einer kosmetischen oder dermatokosmetischen Zusammensetzung als Hauptmittel zur Verringerung des Wasserverlustes der Haut und/oder der Keratinfasern.